# EUROPÄISCHE PATENTSCHRIFT

(11) **EP 2 146 956 B1**
(45) Veröffentlichungstag und Bekanntmachung des Hinweises auf die Patenterteilung: **02.04.2014**
(21) Anmeldenummer: 08758352.2
(22) Anmeldetag: 29.04.2008
(51) Int. Cl.: C07C 303/14, C07C 303/44, C07C 309/04

(54) **VERFAHREN ZUR ISOLIERUNG KONZENTRIERTER PARAFFINSULFONSÄUREN**
METHOD FOR ISOLATING CONCENTRATED PARAFFIN SULFONIC ACIDS
PROCÉDÉ D'ISOLEMENT D'ACIDES SULFONIQUES PARAFFINIQUES CONCENTRÉS

(30) Priorität: 03.05.2007 DE 102007020697
(43) Veröffentlichungstag der Anmeldung: 27.01.2010
(73) Patentinhaber: Clariant Finance (BVI) Limited, Road Town, Tortola (VG)
(72) Erfinder: REINHARDT, Gerd, 65779 Kelkheim (DE); NAUMANN, Peter, 65232 Taunusstein (DE); CUYPERS, Lars, 81369 München (DE)
(74) Vertreter: Ackermann, Joachim
(86) Internationale Anmeldenummer: PCT/EP2008/003453
(87) Internationale Veröffentlichungsnummer: WO 2008/135198

(56) Entgegenhaltungen:
- EP-A- 0 158 235
- EP-A- 0 268 224
- EP-A- 0 402 978
- GB-A- 1 204 514

## Beschreibung

Die vorliegende Erfindung betrifft ein Verfahren zur Isolierung konzentrierter Paraffinsulfonsäuren aus den bei der Sulfoxidation von (C₁₀-C₂₂) Paraffinen erhaltenen Reaktionsgemischen, welches dadurch gekennzeichnet ist, dass man nach weitestgehender Abtrennung von Schwefelsäure das Restparaffin und Wasser aus dem Reaktionsgemisch durch Vakuumdestillation abtrennt.

Salze der Paraffinsulfonsäuren werden seit vielen Jahren in der Wasch- und Reinigungsmittelindustrie aber auch im petrochemischen Sektor verwendet. Die großtechnische Herstellung von Paraffinsulfonsäuren erfolgt durch Sulfoxidation langkettiger (C₁₀-C₂₂) Paraffine (z. B. DE 0 910 165). Um aus dem bei der Sulfoxidation anfallenden Reaktionsgemisch technisch brauchbare Produkte mit tensidischen Eigenschaften zu gewinnen, müssen gelöstes Schwefeldioxid, gebildete Schwefelsäure und überschüssige Paraffine möglichst quantitativ entfernt werden.

Hierfür werden in der Literatur verschiedene Verfahren beschrieben. So wird beispielsweise in DE 0 907 052 ein Methanol-Wasser-Verfahren beschrieben, bei dem Paraffin durch Extraktion mit Kohlenwasserstoffen wie Trimethylpentan abgetrennt wird. DE 0 910 165 schützt ein Verfahren, bei dem Paraffin nach der Sulfoxidation durch Zusatz von Methanol abgetrennt wird. Nach Zugabe von Isooctan und Eindampfen der Lösung bei 120 °C wurde eine Produktphase isoliert, welche sich aus 70 bis 75 % Paraffinsulfonsäuren, 7-8 % Schwefelsäure und Wasser zusammensetzt. In DE 2 139 477 erfolgt die Paraffinabtrennung durch Zugabe von Methanol. Anschließend wird der Gehalt an Schwefelsäure durch Zugabe von Heptanol auf ein tolerierbares Maß reduziert. Auch in DE 2 730 245 wird Paraffin durch Zugabe von Methanol abgetrennt, während in einem weiteren Schritt die Schwefelsäure durch Versetzen der Reaktionsmischung mit Butylether und Phasentrennung entfernt wird. Die isolierte Produktphase besteht aus 65 % Paraffinsulfonsäure, 11 % Schwefelsäure und 24 % Wasser.

Eine weitere Möglichkeit der Isolierung von Paraffinsulfonsäuren wird in DE 3 301 727 beschrieben, wobei nach Abtrennung der Schwefelsäure die Paraffinsulfonsäure mit Glykol extrahiert wird. Die auf diese Weise erhaltene Produktphase besteht zu 50 bis 60 % aus Paraffinsulfonsäure, 20 bis 30 % Sulfonsäure-Glykol-Ester, 10 bis 20 % Glykol und Wasser. Nachteilig bei diesem Extraktionsverfahren ist zum einen das aufwendige Lösungsmittelrecycling, die Bildung unerwünschter Sulfonsäureestern und die niedrige Konzentration der erhaltenen Paraffinsulfonsäuren.

Eine weitere Möglichkeit zur Abtrennung und Reinigung von Paraffinsulfonsäuren wird in EP 0 430 352 geschützt. Hierbei wird zunächst das Sulfoxidationsgemisch zur Entfernung von Paraffin mit superkritischem Kohlendioxid extrahiert, anschließend mit konzentrierter Schwefelsäure versetzt und einer Extraktion mit Cyclohexan unterzogen. Auch bei diesem Verfahren kann auf den Einsatz eines Lösungsmittels nicht verzichtet werden. Außerdem erfordert die Handhabung von superkritischem Kohlendioxid spezielle Apparaturen.

In FR 1 603 096 wird die Behandlung des Reaktionsgemisches mit sauerstoffgesättigtem Wasser bei 60 bis 180 °C, Aufkonzentrieren und Abtrennen der Schwefelsäure vorgeschlagen, wobei eine etwa 40 %ige Paraffinsulfonsäure erhalten wird.

In DE 2 045 087 wird eine Dampfdestillation bei 80 bis 250 °C und 50 bis 760 mm Hg, gefolgt von der Abtrennung der Schwefelsäure empfohlen, wobei als Produkt eine wässrige Paraffinsulfonsäure mit Aktivgehalten von 70 bis 80 % erhalten wird. Als nachteilig erweist sich bei diesem Verfahren die thermische Dampfbehandlung der Paraffinsulfonsäure in Gegenwart der Schwefelsäure, sowie die benötigten Dampfmengen, die einer Kommerzialisierung des Verfahrens entgegenstehen.

In der überwiegenden Anzahl der beschriebenen Verfahren wird die Abtrennung der Schwefelsäure von der Paraffinsulfonsäure erst auf der letzten Reaktionsstufe vorgenommen, so dass es, Insbesondere bei thermischen Verfahren, zu unerwünschten Nebenreaktionen, hervorgerufen durch Schwefelsäure kommt, die die Qualität der Paraffinsulfonsäuren erheblich beeinträchtigen können.

Keines der beschriebenen Verfahren zur Isolierung längerkettiger Paraffinsulfonsäuren hat sich bislang technisch durchsetzen können, weil entweder der Anteil der abgetrennten Schwefelsäure und/oder Paraffine zu gering, der Aufwand zur destillativen Abtrennung des verwendeten Lösungsmittels zu hoch, der Aktivgehalt der erhaltenen Paraffinsulfonsäure < 80 % oder ihre Qualität ungenügend war.

In EP 0 131 913 wird darauf hingewiesen, dass sich die im sauren Reaktionsgemisch enthaltenen Paraffinsulfonsäuren bei Temperaturen oberhalb von 100 °C zersetzen, wobei eine beginnende Zersetzung bereits ab 50 °C vermutet wird.

Aufgabe der Erfindung war es, aus Sulfoxidationsgemischen konzentrierte Paraffinsulfonsäuren zu isolieren, die einen geringen Restparaffingehalt und einen geringen Gehalt an Schwefelsäure aufweisen.

Die EP-A 0 402 978 beschreibt bereits ein Verfahren zur Aufreinigung von Paraffinsulfonsäuren mit den Schritten Dekantieren, Abdestillieren von Wasser und Abtrennen von Restparaffin durch nachfolgende Extraktion mit superkritischem CO₂. Das Entfernen von Restparaffin durch Vakuumdestillation ist in der Schrift nicht offenbart.

Die EP-A 0 268 224 beschreibt die Aufreinigung von mit Alkoholen vermischtem Alkansulfonat-Rohprodukt in einer Zweistufendestillation in zwei Fallfilmverdampfern. Das Entfernen von Restparaffin durch Vakuumdestillation ist in der Schrift ebenfalls nicht offenbart.

Die EP-A 0158 235 beschreibt das Abtrennen von Schwefelsäure von einem bei der Sulfonierung von Paraffinen anfallendem Reaktionsgemisch. Dabei wird die Schwefelsäure als Sulfat ausgefällt und abgetrennt. Eine destillative Aufarbeitung ist in der Schrift nicht offenbart.

Überraschenderweise wurde nun gefunden, dass längerkettige Paraffinsulfonsäuren thermisch beständig sind und destillativ von Paraffinen abgetrennt werden können, wenn der Großteil der Schwefelsäure vor dem Destillationsschritt abgetrennt worden ist.

Gegenstand der Erfindung ist ein Verfahren zur Isolierung von konzentrierten Paraffinsulfonsäuren aus bei der Sulfoxidation von (C₁₀ - C₂₂) n-Paraffinen anfallenden Reaktionsgemischen, die durch Phasentrennnung von einem Großteil der Schwefelsäure und des Paraffins befreit wurden und aus 10 bis 50 % Paraffinsulfonsäuren, 30 bis 70 % n-Paraffinen, 0 bis 40 % Wasser und 0 bis 5 % Schwefelsäure bestehen, dadurch gekennzeichnet, dass man Paraffin und ggf. Restwasser aus dem Reaktionsgemisch durch Vakuumdestillation abtrennt und die Vakuumdestillation bei einer Temperatur von 40 bis 150 °C und einem Druck von 10⁻⁴ bis 1 mbar durchgeführt wird. Die so erhaltene aufkonzentrierte Paraffinsulfonsäure kann anschließend gebleicht und/oder zur Gewinnung der entsprechenden Paraffinsulfonate (Alkansulfonate) mit Na- oder K-hydroxid neutralisiert werden.

Als Ausgangsmaterial für das Verfahren gemäß der vorliegenden Erfindung eignen sich die Reaktionsgemische, wie sie nach an sich bekannten Verfahren, beispielsweise gemäß DE 0 735 096, DE 0 910165 oder DE 1 139 116, durch Sulfoxidation von n-Paraffinen gewonnen werden. Aus diesen Reaktionsgemischen wird von der Destillation gemäß der vorliegende Erfindung zunächst nach an sich bekannten Verfahren ein Großteil des überschüssigen n-Paraffins und der entstandenen Schwefelsäure mittels Phasentrennung abgetrennt. Das verbleibende Reaktionsgemisch, bestehend aus Paraffinsulfonsäure, Restparaffin, Wasser und geringen Mengen an Restschwefelsäure dient dann als Ausgangsmaterial für das erfindungsgemäße Verfahren.

Bevorzugt setzt sich das Ausgangsmaterial aus 12 bis 40 % Paraffinsulfonsäuren, 40 bis 60 % n-Paraffinen, 15 bis 40 % Wasser und 0 bis 3 % Schwefelsäure zusammen. Falls gewünscht, kann zur vollständigen Schwefelsäureentfernung das Ausgangsmaterial für das Verfahren der vorliegenden Erfindung wie in Beispiel 2 beschrieben mit Erdalkalisalzen, vorzugsweise Calciumcarbonat oder Calciumhydroxid, versetzt und die ausgefallenen Erdalkalisulfate abfiltriert werden.

Gegebenenfalls kann das verwendete Ausgangsmaterial vor der Vakuumdestillation in einer Entgasungsstufe noch von leichtsiedenden Substanzen, wie z. B. Wasser, befreit werden. Dies kann, wie dem Fachmann bekannt, z. B. mit Hilfe eines Fallfilmverdampfers oder einer Flashbox im Temperaturbereich von 20 bis 120 °C, vorzugsweise 40 bis 80 °C unter reduziertem Druck geschehen.

Zur Gewinnung der freien Paraffinsulfonsäuren wird das vorher beschriebenen Ausgangsmaterial oder die bereits weitgehend entwässerte Variante einer Vakuumdestillation unterworfen. Die Vakuumdestillation wird bei Temperaturen von 40 bis 150 °C, bevorzugt 60 bis 100 °C, und Drücken von 10⁻⁴ bis 1 mbar, bevorzugt 10⁻² bis 0,1 mbar, durchgeführt. Um eine übermäßige thermische Belastung des Produktes zu vermeiden, werden kurze Verweilzeiten während der Trennung angestrebt. Weiterhin hat es sich als vorteilhaft erwiesen, den Reaktionssumpf nach erfolgter Abtrennung des Paraffins möglichst schnell auf < 80 °C, vorzugsweise < 60 °C abzukühlen, um ein farblich helles Produkt zu erhalten. Unter bestimmten Reaktionsbedingungen ist nicht auszuschließen, dass sich während der thermischen Belastung des Reaktionsgemisches Anhydride, Ester etc. als Nebenprodukte in geringen Mengen bilden. Um diese Verbindungen in die freie Paraffinsulfonsäure zu überführen, hat es sich als vorteilhaft erwiesen, den Reaktionssumpf (Paraffinsulfonsäure) nach der thermischen Trennung mit 0,1 bis 5 % Wasser zu versetzen.

Als Apparatur für die Vakuumdestillation eignen sich Verdampfer, wie sie dem Fachmann bekannt sind, z. B. Dünnschichtverdampfer, Fallfilmverdampfer oder Kurzwegverdampfer, wie sie zur Zeit z. B. von den Firmen Buss-SMS-Canzler, UIC, Gig Karassek oder Gea Wiegand angeboten werden. Aus Korrosionsgründen sind besondere Ansprüche an die Werkstoffe der Verdampfereinheit zu stellen, vorzugsweise werden Apparate aus Hastelloy, Tantal, Glas, Emaille oder ähnlich korrosionsstabilen Werkstoffen oder Beschichtungen verwendet.

Zum Erzeugen des benötigten Vakuums können z. B. Flüssigkeitsring-, Drehschieber-, Kreiskolben-, Sperrschieber-, Wälzkolben-, Klauen-, Scroll-, Membran-, Hubkolben-, Turbomolekular-, Öldiffusions- oder Öldampfstrahlpumpen, aber auch gasbindende Vakuumpumpen oder geeignete Kombinationen von verschiedenen Vakuumpumpen eingesetzt werden.

Je nach Destillationsbedingungen kann die isolierte Paraffinsulfonsäure eine dunkle Farbe aufweisen. Gegebenfalls kann die gewonnene Paraffinsulfonsäure deshalb im Anschluss an die Destillation mit Bleichmitteln wie z. B. Wasserstoffperoxid behandelt werden. Hierbei werden 0,1 bis 10 % Wasserstoffperoxyd bei 10 bis 60 °C mit der Paraffinsulfonsäure vermischt. Die Reaktionszeit beträgt je nach Temperatur 10 min bis 6 h. Es hat sich als vorteilhaft erwiesen die gebleichte Paraffinsulfonsäure durch Zugabe von 1 bis 15 % Wasser zu stabilisieren. Die gebleichte Paraffinsulfonsäure kann anschließend in ein Salz überführt, einer Wasch- und Reinigungsmittelformulierung zugesetzt oder als freie Säure in konzentrierter oder verdünnter Form verwendet werden.

Ein wesentlicher Vorteil der erfindungsgemäßen Arbeitsweise ist darin zu sehen, dass es mit dem beschriebenen Verfahren gelingt, helle, geruchs-, schwefelsäure- und paraffinarme freie Paraffinsulfonsäuren wirtschaftlich zu isolieren.

Man erhält auf diese Weise Paraffinsulfonsäuren mit einem Gehalt an Aktivsubstanz von über 85 %, vorzugsweise über 90 %. Der Restparaffingehalt ist geringer als 5 %, vorzugsweise geringer als 3 % und der Gehalt an Schwefelsäure liegt unter 5 Gew.-%, vorzugsweise unter 3 Gew.-%

### Beispiele

### Beispiel 1

In einem 1 l Loop-Reaktor wurden 1 l/h C₁₄/C₁₇-Paraffin mit Wasser vermischt und mit 670 Nl/h Schwefeldioxid und 330 Nl/h Sauerstoff begast. Das Reaktionsgemisch wurde mit einer Mitteldruckquecksilberlampe (TQ 150 Heraeus) bei einer Temperatur von 38 °C bestrahlt bis im Reaktionsgemisch 0,6 % Wasser, 93,8 % C₁₄/C₁₇-Paraffin, 4,1 % C₁₄/C₁₇-Paraffinsulfonsäure und 1,5 % H₂SO₄ vorlagen. Aus dieser Mischung trennte sich Schwefelsäure ab. Die obere Phase wurde isoliert und mit Wasser versetzt, dass sich eine obere Phase aus Paraffin abschied. Die untere Phase bestand aus 29,1 % C₁₄/C₁₇-Paraffinsulfonsäure, 49,0 % C₁₄/C₁₇-Paraffin, 20,5 % Wasser und 1,4 % Schwefelsäure.

Die untere Phase wurde bei 1,5 mbar und 70 °C mit Hilfe eines Fallfilmverdampfers entwässert, wobei das Zwischenprodukt im Sumpf aus 38 % C₁₄/C₁₇-Paraffin-sulfonsäure, 60 % C₁₄/C₁₇-Paraffin, 0,1 % Wasser und 1,8 % Schwefelsäure bestand. Das Zwischenprodukt wurde im Anschluss bei 0,05 mbar und 100 °C in einem Kurzwegverdampfer destilliert, wobei ein dunkelbrauner Produktsumpf der Zusammensetzung 94,2 % C₁₄/C₁₇-Paraffin-sulfonsäure, 1,2 % C₁₄/C₁₇-Paraffin, < 0,1 % Wasser und 1,2 % Schwefelsäure gewonnen wurde.

### Beispiel 2

Es wurde analog Beispiel 1 verfahren und das Reaktionsgemisch aufgearbeitet. Nach Abtrennung des Paraffins und der Hauptmenge der Schwefelsäure wurde eine Phase folgender Zusammensetzung erhalten:
14,8 % C₁₄/C₁₇-Paraffinsulfonsäure, 49,1 % C₁₄/C₁₇-Paraffin, 34,9 % Wasser und 1,2 % Schwefelsäure.
1.000 g der Produktphase wurden mit 13 g Calciumcarbonat versetzt und vier Stunden bei Raumtemperatur gerührt. Das ausfallende Calciumsulfat wurde abfiltriert. Das Filtrat bestand aus 14,9 % C₁₄/C₁₇-Paraffinsulfonsäure, 49,5 % C₁₄/C₁₇-Paraffin, 35,2 % Wasser und 0,4 % Schwefelsäure.
Das Filtrat wurde bei 1,5 mbar und 70 °C mit Hilfe eines Fallfilmverdampfers entwässert, wobei das Zwischenprodukt im Sumpf aus 23,7 % C₁₄/C₁₇-Paraffinsulfonsäure, 75,4 % C₁₄/C₁₇-Paraffin, 0,3 % Wasser und 0,6 % Schwefelsäure bestand.
Das Zwischenprodukt wurde im Anschluss bei 0,1 mbar und 100 °C in einem Kurzwegverdampfer destilliert, wobei ein rotbrauner Produktsumpf der Zusammensetzung 94,6 % C₁₄/C₁₇-Paraffinsulfonsäure, 2,8 % C₁₄/C₁₇-Paraffin, < 0,1 % Wasser und 2,4 % Schwefelsäure gewonnen wurde.

### Beispiel 3

Zur Destillation wurde ein Reaktionsgemisch der Zusammensetzung 36,4 % C₁₄/C₁₇-Paraffinsulfonsäure, 58,2 % C₁₄/C₁₇-Paraffin, 4,4 % Wasser und 1,9 % Schwefelsäure verwendet.

Die thermische Stabilität des Reaktionsgemisches wurde vorab mit der Differenzthermoanalyse (DTA) im geschlossenen Tiegel mit einer Heizrate von 3 K/min ausgeprüft. Um katalytische Wandeinflüsse bei der Messung zu verhindern, wurden ausschließlich Glastiegel verwendet. Die Probenvorbereitung erfolgte unter Luft, der Effekt ist jedoch vernachlässigbar, da der Energieinhalt einer möglichen Oxidationsreaktion aufgrund des überlagerten Sauerstoffpartialdrucks mit rund -20 J/g ohne Bedeutung ist. Die Untersuchung der Probe zeigte im untersuchten Temperaturbereich von 25 bis 400 °C eine schwach exotherme Zersetzungsreaktion ab 280 °C. Diese kann aufgrund der geringen Energiefreisetzung von -50 J/g und der geringen Reaktionsleistung nicht zu einer sich kritisch selbstbeschleunigenden Zersetzungsreaktion führen.

Das Reaktionsgemisch wurde bei 3*10⁻³ mbar und 110 °C in einem Glasdünnschichtverdampfer destilliert. Im Destillationssumpf wurde ein dunkelbraunes Produkt der Zusammensetzung
93,5 % C₁₄/C₁₇-Paraffinsulfonsäure, 1,5 % C₁₄/C₁₇-Paraffin, < 0,1 % Wasser und 4,9 % Schwefelsäure erhalten.

### Beispiel 4

100 g dunkelbraune Alkansulfonsäure der Zusammensetzung
93,5 % C₁₄/C₁₇-Paraffinsulfonsäure, 1,5 % C₁₄/C₁₇-Paraffin, < 0,1 % Wasser und 4,9 % Schwefelsäure wurden bei Raumtemperatur mit 3 g Wasserstoffperoxid (30 %ig) versetzt und 4 Stunden bei Raumtemperatur gerührt. Dann wurden 15 g destilliertes Wasser zugegeben. Das Produkt war eine hellgelbe Flüssigkeit mit der Zusammensetzung 79,2 % C₁₄/C₁₇-Paraffinsulfonsäure,
1,3 % C₁₄/C₁₇-Paraffin, 15,3 % Wasser und 4,2 % Schwefelsäure.

## Patentansprüche

1. Verfahren zur Isolierung von konzentrierten Paraffinsulfonsäuren aus bei der Sulfoxidation von (C₁₀ - C₂₂) n-Paraffinen anfallenden Reaktionsgemischen, die durch Phasentrennung von einem Großteil der Schwefelsäure und des Paraffins befreit wurden und aus 10 bis 50 % Paraffinsulfonsäuren, 30 bis 70 % n-Paraffinen, 0 bis 40 % Wasser und 0 bis 5 % Schwefelsäure bestehen, **dadurch gekennzeichnet, dass** man Paraffin und ggf. Restwasser aus dem Reaktionsgemisch durch Vakuumdestillation abtrennt und die Vakuumdestillation bei einer Temperatur von 40 bis 150 °C und einem Druck von 10⁻⁴ bis 1 mbar durchgeführt wird.

2. Verfahren nach Anspruch 1, **dadurch gekennzeichnet, dass** das Ausgangsmaterial für die Vakuumdestillation aus 12 bis 40 % Paraffinsulfonsäuren, 40 bis 60 % n-Paraffinen, 15 bis 40 % Wasser und 0 bis 3 % Schwefelsäure besteht.

3. Verfahren nach Anspruch 1, **dadurch gekennzeichnet, dass** die Vakuumdestillation bei einer Temperatur von 50 bis 150 °C durchgeführt wird.

4. Verfahren nach Anspruch 1, **dadurch gekennzeichnet, dass** die Vakuumdestillation bei einer Temperatur von 80 bis 120 °C durchgeführt wird.

5. Verfahren nach Anspruch 1, **dadurch gekennzeichnet, dass** die Vakuumdestillation bei einem Druck von 10⁻³ mbar bis 1 mbar durchgeführt wird.

6. Verfahren nach Anspruch 1, **dadurch gekennzeichnet, dass** die Vakuumdestillation bei einem Druck von 10⁻² mbar bis 0,1 mbar durchgeführt wird.

7. Verfahren nach Anspruch 1, **dadurch gekennzeichnet, dass** die Vakuumdestillation in einem Dünnschichtverdampfer durchgeführt wird.

8. Verfahren nach Anspruch 1, **dadurch gekennzeichnet, dass** die Vakuumdestillation in einem Kurzwegverdampfer durchgeführt wird.

9. Verfahren nach Anspruch 1, **dadurch gekennzeichnet, dass** das Ausgangsmaterial vor der Vakuumdestillation entwässert wird.

10. Verfahren nach Anspruch 1, **dadurch gekennzeichnet, dass** die Schwefelsäure im Ausgangsmaterial vor der Vakuumdestillation mit Erdalkalisalzen neutralisiert wird und die ausgefallenen Erdalkalisulfate vom Ausgangsmaterial abgetrennt werden.

## Claims

1. A method for isolating concentrated paraffin sulfonic acids from reaction mixtures that are produced during the sulfoxidation of (C₁₀-C₂₂) n-paraffins, from which a major part of the sulfuric acid and the paraffin has been removed by phase separation and which consist of 10 to 50% paraffin sulfonic acids, 30 to 70% n-paraffins, 0 to 40% water and 0 to 5% sulfuric acid, which comprises removing paraffin and optionally residual water from the reaction mixture by vacuum distillation and carrying out the vacuum distillation at a temperature from 40 to 150°C and a pressure from 10⁻⁴ to 1 mbar.

2. The method as claimed in claim 1, wherein the starting material for the vacuum distillation consists of 12 to 40% paraffin sulfonic acids, 40 to 60% n-paraffins, 15 to 40% water and 0 to 3% sulfuric acid.

3. The method as claimed in claim 1, wherein the vacuum distillation is carried out at a temperature from 50 to 150°C.

4. The method as claimed in claim 1, wherein the vacuum distillation is carried out at a temperature from 80 to 120°C.

5. The method as claimed in claim 1, wherein the vacuum distillation is carried out at a pressure from 10⁻³ mbar to 1 mbar.

6. The method as claimed in claim 1, wherein the vacuum distillation is carried out at a pressure from 10⁻² mbar to 0.1 mbar.

7. The method as claimed in claim 1, wherein the vacuum distillation is carried out in a thin-film evaporator.

8. The method as claimed in claim 1, wherein the vacuum distillation is carried out in a short-path evaporator.

9. The method as claimed in claim 1, wherein the starting material is dewatered prior to the vacuum distillation.

10. The method as claimed in claim 1, wherein the sulfuric acid in the starting material is neutralized with alkaline earth metal salts prior to the vacuum distillation and the alkaline earth metal sulfates that are produced are separated off from the starting material.

## Revendications

1. Procédé pour l'isolement d'acides paraffinesulfoniques concentrés à partir de mélanges réactionnels obtenus lors de la sulfoxydation de n-paraffines en (C₁₀-C₂₂), qui ont été libérés par séparation de phases d'une grande partie de l'acide sulfurique et de la paraffine et qui sont constitués par 10 à 50% d'acides paraffinesulfoniques, 30 à 70% de n-paraffines, 0 à 40% d'eau et 0 à 5% d'acide sulfurique, **caractérisé en ce qu'**on sépare la paraffine et le cas échéant l'eau résiduelle du mélange résiduel par distillation sous vide et la distillation sous vide est réalisée à une température de 40 à 150°C et une pression de 10⁻⁴ à 1 mbar.

2. Procédé selon la revendication 1, **caractérisé en ce que** le matériau de départ pour la distillation sous vide est constitué par 12 à 40% d'acides paraffinesulfoniques, 40 à 60% de n-paraffines, 15 à 40% d'eau et 0 à 3% d'acide sulfurique.

3. Procédé selon la revendication 1, **caractérisé en ce que** la distillation sous vide est réalisée à une température de 50 à 150°C.

4. Procédé selon la revendication 1, **caractérisé en ce que** la distillation sous vide est réalisée à une température de 80 à 120°C.

5. Procédé selon la revendication 1, **caractérisé en ce que** la distillation sous vide est réalisée à une pression de 10⁻³ mbar à 1 mbar.

6. Procédé selon la revendication 1, **caractérisé en ce que** la distillation sous vide est réalisée à une pression de 10⁻² mbar à 0,1 mbar.

7. Procédé selon la revendication 1, **caractérisé en ce que** la distillation sous vide est réalisée dans un évaporateur à couche mince.

8. Procédé selon la revendication 1, **caractérisé en ce que** la distillation sous vide est réalisée dans un évaporateur instantané.

9. Procédé selon la revendication 1, **caractérisé en ce que** le matériau de départ est déshydraté avant la distillation sous vide.

10. Procédé selon la revendication 1, **caractérisé en ce que** l'acide sulfurique dans le matériau de départ est neutralisé avant la distillation sous vide par des sels de métal alcalino-terreux et les sulfates de métal alcalino-terreux précipités sont séparés du matériau de départ.
